(19) Europäisches Patentamt
European Patent Office
Office européen des brevets

(11) Numéro de publication: **0 528 925 B1**

(12) **FASCICULE DE BREVET EUROPEEN**

(45) Date de publication de fascicule du brevet: **28.09.94**

(51) Int. Cl.5: **C12N 15/74**, C12P 21/00, C12N 15/70

(21) Numéro de dépôt: **91909570.3**

(22) Date de dépôt: **23.04.91**

(86) Numéro de dépôt internationale :
**PCT/FR91/00333**

(87) Numéro de publication internationale :
**WO 91/16439 (31.10.91 91/25)**

(54) **NOUVEAUX VECTEURS DE CLONAGE ET/OU D'EXPRESSION, PROCEDE DE PREPARATION ET LEUR UTILISATION.**

(30) Priorité: **24.04.90 FR 9005185**

(43) Date de publication de la demande:
**03.03.93 Bulletin 93/09**

(45) Mention de la délivrance du brevet:
**28.09.94 Bulletin 94/39**

(84) Etats contractants désignés:
**AT BE CH DE DK ES FR GB GR IT LI LU NL SE**

(56) Documents cités:
**US-A- 4 590 163**

**JOURNAL OF BIOTECHNOLOGY, vol. 4, 1986, Elsevier Science Publishers B.V., P.N. Saurugger et al.: "Mapping and cloning of the par-region of broad-host-range plasmid RP4", pages 333-343, see abstract, page 334, paragraph 5; page 339 last paragraph; page 340, paragraphs 1,2; discussion**

(73) Titulaire: **RHONE-POULENC BIOCHIMIE**
**20 avenue Raymond Aron**
**F-92160 Antony Cedex (FR)**

(72) Inventeur: **CAMERON, Béatrice**
**118, rue d'Assas**
**F-75006 Paris (FR)**
Inventeur: **CROUZET, Joel**
**48-52, rue des Meuniers**
**F-75012 Paris (FR)**
Inventeur: **LEVY SCHIL, Sophie**
**2, rue de Monttessuy**
**F-75007 Paris (FR)**

(74) Mandataire: **Savina, Jacques et al**
**RHONE-POULENC RORER S.A.,**
**Direction des Brevets,**
**20 avenue Raymond Aron**
**F-92160 Antony Cédex (FR)**

## Description

La présente invention concerne de nouveaux vecteurs de clonage et/ou d'expression à large spectre d'hôtes chez les bactéries gram-négatives. Elle concerne également l'utilisation de ces vecteurs, notamment dans la production de protéines recombinantes ou de métabolites, ou dans des réactions de biotransformations, ainsi que les cellules hôtes contenant de tels vecteurs.

Les progrès recents de la biologie moléculaire ont conduit au developpement de nouveaux vecteurs, utilisables dans les procédés du génie génétique, possèdant un large spectre d'hôtes. Ces vecteurs s'opposent à ceux ayant une spécificité d'hôtes étroite, tels que ColE1, par le caractère ubiquitaire, au niveau taxonomique, des hôtes chez lesquels ils se répliquent. Ce caractère est très prononcé pour certains plasmides qui se répliquent chez presque toutes les bactéries gram-négatives. De tels plasmides présentent la particularité de pouvoir être utilisés chez des bactéries telles qu'Escherichia coli pour faire les constructions nécessaires, pour être ensuite directement introduits chez l'hôte gram-négatif que l'on a choisi. Une autre caractéristique de ces vecteurs réside dans la propriété qu'ils ont soit de se mobiliser soit d'être mobilisés pour le transfert conjugatif d'une bactérie donatrice vers une bactérie réceptrice. Ces transferts conjugatifs se font en général avec une fréquence très élevée (entre 1 et $10^{-2}$ ), largement supérieure aux systèmes de transformation connus.

Les plasmides à large spectre d'hôtes chez les bactéries gram-négatives decrits dans la littérature appartiennent aux groupes d'incompatibilité C, N, P, Q et W (incC, incN, incP, incQ et incW). Parmi ceux-ci, les plasmides des groupes d'incompatibilité P et Q sont ceux qui sont le mieux étudiés et ceux pour lesquels un nombre élevé de dérivés ont été construits (Schmidhauser et al., 1988). Ce sont aussi ceux qui sont presque exclusivement utilisés. A cet égard, il est décrit que ces plasmides se répliquent chez toutes les bactéries gram-négatives qui sont étudiées sauf Myxococcus xanthus, Bradyrhizobium japonicum et les bactéries du genre Bacteroïdes (Kües et Stahl, 1989). Les plasmides du groupe d'incompatibilité W sont utilisés de manière marginale, d'une part parce qu'ils présentent moins d'avantages que les plasmides des groupes d'incompatiblité P et Q et d'autre part parce qu'ils sont moins bien connus.

Le groupe d'incompatibilité P est divisé en deux sous groupes: incPα et incPβ, les plasmides les plus étudiés étant : RK2, RP4 (qui sont en fait deux isolats indépendants du même plasmide) et R751. Ces plasmides présentent la caractéristique d'être autoconjugatifs, c'est à dire qu'ils portent les fonctions de conjugaisons (tra et mob). De plus, ce sont de grands plasmides (RK2 a une taille de 60 kb) à faible nombre de copies (RK2 a un nombre de copies compris entre 4 et 7 chez E. coli).

La réplication de ces plasmides a été étudiée en détail par Kües et Stahl. Ainsi, on sait que l'origine de réplication oriV est composée de (i) 8 segments de 17 pb, (ii) un promoteur putatif entouré par des sites de fixation de la protéine DnaA de E. coli et un site putatif de fixation de la protéine IHF de E. coli, (iii) une séquence de 9 pb riche en AT suivie d'un site de fixation de la protéine DnaA, puis (iv) une région riche en GC. L'origine de réplication associée au gène trfA, qui est traduit en deux protéines A1 et A2 codées par la même phase mais qui sont initiées à des codons différents, constitue un réplicon qui a la même ubiquité que RK2. Suivant les hôtes, soit A2 semble nécessaire pour la réplication soit A1 et A2 sont toutes les deux indispensables (Thomas, 1986). La réplication de ces plasmides est contrôlée par un réseau de régulation complexe, dans lequel interviennent les gènes kil et kor. Les gènes kor pour (kil-override) antagonisent les effets léthaux de gènes kil (kilA, kilB, kilC et kilD), et régulent négativement l'expression, de trfA. Cinq gènes kor ont été identifiés (korA, korB, korC, korE et korF). Le réseau de régulation de ces gènes entre eux et avec trfA est reconnu comme permettant l'adaptation du réplicon RK2 à l'hôte chez lequel il se réplique (Kües et Stahl, 1989). Schmidhauser et Helinski (1985) ont aussi montré que la délétion de certains de ces gènes résultait chez certains hôtes dans une plus faible stabilité ségrégationnelle .

Il apparaît donc que tous ces gènes sont nécessaires pour observer la meilleure stabilité possible des dérivés de RK2 ou RP4.

Plusieurs dérivés de RK2 ont été construits en tant que vecteurs multihôtes. Ce sont des plasmides qui ont conservé le gêne de résistance à la tétracycline du vecteur de départ puisque celui-ci est situé près de l'origine de réplication (oriV). Il s'agit par exemple des plasmides pRK290, pRK404, pRK415 (Ditta et al, 1980 et 1985, Keen et al, 1988, Schmidhauser et al., 1988). Parmi ceux-ci, on citera plus particulièrement le plasmide pRK290 (Ditta et al., 1980), qui montre des propriétés de grande stabilité chez bon nombre de bactéries gram-négatives (Schmidhauser et Helinski, 1985). Ce plasmide se mantient de manière stable chez Escherichia coli, Azotobacter, Pseudomonas putida, Rhizobium meliloti, Rhodopseudomonas sphaeroïdes. Toutefois chez d'autres bactéries gram-négatives, ce même plasmide n'est pas aussi stable; il s'agit d'Agrobacterium tumefaciens, d'Acetinobacter, de Caulobacter et de Pseudomonas aeruginosa - (Schmidhauser et Helinski, 1985). Le plasmide pRK290 a une taille de 20 kb, ce qui est grand pour un vecteur de clonage. Il a été obtenu par une série de délétions successives à partir de RK2. Outre les fonctions de transferts (gènes tra), le gène de résistance à la kanamycine et le transposon Tn1, ces délétions ont enlevé les fonctions kilA et kilC, korC et korE (Schmidhauser et Helinski, 1985). Il semble donc qu'il soit possible d'observer une certaine stabilité chez un dérivé de RK2 malgré la perte de certaines fonctions kil et kor. Toutefois, il a été observé que la simple perte de la fonction kilB se traduisait par une diminution nette de la stabilité du plasmide chez plusieurs bactéries gram-négatives, et que tous les dérivés de RK2 testés et ayant une plus petite taille que le pRK290 étaient nettement moins stables (Schmidhauser et Helinski, 1985).

Mis à part les études qui ont mis en lumière l'importance respective des différents déterminants de RK2 impliqués dans le système de régulation de l'expression de trfA, il a été trouvé un fragment pouvant être responsable de la stabilité ségrégationnelle de ces plasmides (Sauruger et al., 1986). Ce fragment, positionné entre la région tra2 et le gène de résistance à la kanamycine, code pour un système de partition (par). Il a été montré que ce fragment était capable de stabiliser chez Escherichia coli les plasmides pBR322 et pACYC177 (Sauruger et al., 1986).

En ce qui concerne les plasmides naturels du groupe d'incompatibilité Q, on peut citer par exemple RSF1010, R1162 et R300B. Ces plasmides ont un nombre de copies compris entre 10 et 60 suivant les microorganismes. La taille de ces plasmides est bien inférieure à celle des plasmides du groupe d'incompatibilité P; en effet celle-ci est inférieure à 10 kb.

Il y a une région nécessaire en cis pour la réplication de ces plasmides qui comprend deux sous régions: (i) une région formée de 3,5 séquences répétées, de 40 pb riche en AT, de 60 pb riche en GC, et d'une boîte putative de fixation de la protéine DnaA de E. coli; (ii) d'autre part une région avec une séquence inversée répétée qui peut former une structure secondaire avec une tige de 40 à 60 pb. L'initiation de la réplication de RSF1010 nécessite la présence de trois protéines codées par le plasmide: RepA, RepB et RepC codées par les gènes repA, repB et repC. RepC reconnaît l'origine de réplication (au niveau des séquences répétées) et régule positivement l'initiation de la réplication; RepA a une activité hélicase; RepB et RepB* (qui correspondent à deux protéines codées par la même phase mais initiées chacune à un codon différent) ont une activité primase RSF1010-spécifique in vitro. La réplication de RSF1010 est dépendante de l'ADN polymérase III et de la gyrase de l'hôte. RSF1010 peut être mobilisé d'une bactérie gram-négative à une autre bactérie gram-négative par les fonctions tra des plasmides des groupes d'incompatiblité Inclα, IncM, IncX et surtout IncP (Derbyshire et Willets, 1987).

Aucune étude concernant la stabilité de RSF1010 et de ses dérivés n'a été décrite à ce jour. De plus, bien que la séquence de RSF1010 soit connue (Scholz et Scherzinger, 1989), aucun déterminant de stabilité de plasmide n'a pu être identifié, aussi bien par une analyse fonctionnelle que par une analyse moléculaire. Il y a toutes les raisons de penser que les plasmides du groupe d'incompatibilité Q ne sont pas aussi stables que ceux du groupe d'incompatiblité P.

En dépit de ces travaux, les plasmides à large spectre d'hôtes connus présentent un certain nombre d'inconvénients, relatifs à leurs performances et à leur usage au niveau industriel.

En particulier, pour une utilisation industrielle, indépendamment de la bactérie hôte, les plasmides vecteurs doivent présenter un certain nombre de propriétés qui correspondent à des contraintes d'exploitation et de biosécurité.

Les contraintes d'exploitation sont liées essentiellement à la stabilité ségrégationnelle du plasmide. En effet il est très souhaitable de pouvoir éviter l'utilisation à l'échelle industrielle d'antibiotiques pour contre sélectionner la perte du plasmide. Dans cette perspective, un vecteur à utilisation industrielle doit posséder une grande stabilité ségrégationnelle. Celle-ci doit être grande sur au moins 25 générations successives qui représentent le nombre de générations nécessaire pour aller de l'état de conservation de la souche recombinée jusqu'à la fin d'un fermenteur industriel de 200 m³ (Stanburry et Whitaker, 1984). En outre ces propriétés de stabilité doivent être voisines pour les dérivés de tels plasmides portant un insert nucléotidique contenant les séquences d'ADN que l'on veut amplifier, exprimer, etc.

Les contraintes de biosécurité imposent par ailleurs un grand confinement biologique aux souches recombinées. Le système de biosécurité de niveau 1 (BL1) décrit dans le "Guidelines for research involving recombinant DNA molecules" du NIH du 7 Mai 1987 correspond aux plus faibles contraintes. Ce système, aussi bien chez Escherichia coli que chez Pseudomonas putida, suppose l'utilisation de plasmides non conjugatifs et non mobilisables. En effet, si par accident le microorganisme recombiné se trouvait relaché en milieu naturel, il est impératif que de tels plasmides ne puissent se transmettre à d'autres organismes (Trevors et al.,1987).

Or, parmi les plasmides à large spectre d'hôtes décrits dans la littérature, aucun ne satisfait pleinement ces conditions. Tout d'abord, une très bonne stabilité ségrégationnelle n'a jamais été atteinte de manière ubiquitaire. En effet, si certains de ces plasmides sont très stables chez certains hôtes, il n'en est pas de même dans toutes les bactéries gram-négatives. De plus, la stabilité de ces plasmides n'a, la plupart du temps, pas été étudiée après insertion d'un fragment d'ADN, contenant par exemple des séquences que l'on veut amplifier ou exprimer. C'est le cas notamment du plasmide pRK290, dont les caractères de stabilité n'ont jamais été étudiés après insertion d'un fragment d'ADN. Par ailleurs, une autre caractéristique des plasmides à large spectre d'hôtes décrits dans l'art antérieur réside dans le fait qu'ils peuvent se mobiliser ou être mobilisés pour le transfert conjugatif d'une bactérie donatrice vers une bactérie réceptrice. C'est également le cas du plasmide pRK290, qui possède le locus mob. Ces plasmides ne sont donc pas compatibles avec les contraintes de biosécurité, puisqu'en cas de rejet accidentel de l'organisme recombiné, on ne peut exclure que le plasmide, avec les séquences d'ADN qu'il porte, ne soit mobilisé, à une fréquence relativement élevée, chez d'autres microorganismes.

Par ailleurs, le manque de stabilité de certains de ces plasmides est également synonyme de faible efficacité dans la production de protéines ou de métabolites, et donc d'intérêt limité dans les procédés de fermentation industriels.

L'un des objets de la présente invention réside précisément dans un vecteur de clonage et/ou d'expression à large spectre d'hôtes, caractérisé en ce qu'il est capable de se répliquer chez presque toutes les bactéries gram-négatives, présente une grande stabilité ségrégationnelle, et est non mobilisable. L'invention a aussi pour objet tous les dérivés de ce vecteur. Au sens de l'invention, les dérivés de ce vecteur comprennent les vecteurs qui, en dépit de quelques altérations ou modifications (délétions, mutations, insertions, ...) portant sur des régions d'une importance relative, conservent les caractéristiques énoncées ci-dessus.

Les vecteurs selon l'invention dérivent des plasmides à large spectre d'hôtes chez les bactéries gram-négatives du groupe d'incompatibilité P. Plus particulièrement, ils possèdent une origine de réplication dérivée d'un plasmide appartenant au groupe d'incompatibilité P. Avantageusement, ils dérivent du plasmide RK2. Ils combinent des propriétés qui n'ont jamais été décrites pour un vecteur, à savoir : un large spectre d'hôtes chez les bactéries gram-négatives, une grande stabilité, et l'absence de fonctions de mobilisation.

La grande stabilité des vecteurs selon l'invention résulte de l'incorporation d'un fragment nucléotidique particulier, possèdant des propriétés stabilisatrices. Notamment, on peut utiliser un fragment particulier provenant du plasmide naturel RP4. Le fragment cloné, appelé fragment par, a été décrit par Saurugger et al, 1986, ainsi que sa capacité à stabiliser certains plasmides chez E.coli. Il a maintenant été trouvé que l'insertion du fragment par s'accompagne d'un gain très important de stabilité, indépendant de l'organisme hôte choisi, et de la nature (gène, promoteur, ...) ou de la taille de l'insert nucléotidique. En effet, la stabilité ségrégationnelle des vecteurs selon l'invention, ainsi que de leurs dérivés, est plus élevée que celle des dérivés de RK2 pouvant servir de vecteurs de clonage, et ceci dans toutes les bactéries gram-négatives testées.

La perte des fonctions de mobilisation est une seconde caractéristique des vecteurs de l'invention. Elle a pour conséquence que, contrairement aux plasmides tels pRK290, les vecteurs selon l'invention ne sont pas mobilisables d'une bactérie gram-négative à une autre. Ils forment donc avec celles-ci des couples hôte-vecteur de classe 1, et sont conformes à la règlementation industrielle. Cette propriété très avantageuse des vecteurs selon la présente invention peut être obtenue notamment par délétion d'une région portant le locus mob.

La troisième propriété des vecteurs selon l'invention réside dans leur capacité à se répliquer chez la majorité des bactéries gram-négatives. Cette propriété est particulièrement avantageuse, puisqu'elle permet en particulier de travailler chez E. coli pour réaliser les constructions désirées, avant d'introduire le vecteur chez l'hôte gram-négatif choisi. Elle permet également de choisir, en fonction de l'utilisation désirée des vecteurs (amplification, expression ..) ou de la nature et de la taille des inserts nucléotidiques, l'hôte le plus approprié.

Dans un mode de réalisation particulier, les vecteurs selon la présente invention possèdent un multisite de clonage avec un certain nombre de sites de clonage uniques, facilitant considérablement l'intégration des inserts nucléotidiques que l'on souhaite amplifier ou exprimer .

Dans un autre mode de réalisation, les vecteurs selon la présente invention portent un marqueur de sélection, tel qu'en particulier, un gène de résistance à un antibiotique. De préference, ils contiennent le gène de résistance à la tétracycline de RK2. Ce gène de résistance permet de sélectionner la présence du plasmide chez presque toutes les bactéries gram-négatives. D'autres gènes de résistance pourront être insérés sur les vecteurs décrits dans la présente invention ainsi que sur leurs dérivés.

Un objet plus particulier de l'invention concerne un vecteur de clonage et/ou d'expression à large spectre d'hôtes chez les bactéries gram-négatives, caractérisé en ce qu'il dérive du plasmide RK2, a une taille inférieure à 20 Kb, contient un fragment d'ADN portant la région par du plasmide RP4, ne contient pas de locus mob, et eventuellement, contient un multisite de clonage et un marqueur de sélection.

Les vecteurs selon la présente invention peuvent être utilisés pour amplifier ou exprimer des séquences d'ADN données. Un objet de l'invention concerne donc les vecteurs tels que définis ci-dessus contenant un fragment d'ADN recombinant.

Plus particulièrement, l'ADN recombinant contient, sous la dépendance de séquences de régulation de l'expression génétique, un ou plusieurs gènes de structure codant pour un ou plusieurs polypeptides donnés. Toujours selon l'invention, les gènes de structure peuvent être sous la dépendance de leurs propres signaux de régulation, ou de signaux de régulation différents. Dans un mode de réalisation préféré, les séquences de régulation de l'expression génétique sont constituées par un ou plusieurs élements choisis parmi les promoteurs, les terminateurs, les peptides signaux, et les sites de fixation des ribosomes.

Les gènes de structure qui codent pour un polypeptide peuvent notamment être choisis parmi les gènes qui codent pour des enzymes, des hormones, ou des facteurs de croissance. De préference, ils sont choisis parmi le gène de structure de l'albumine, du tPA, du TIMP, des interleukines, des apolipoprotéines, et des interférons.

Dans un mode particulier de réalisation de l'invention, le ou les gènes de structure sont des gènes intervenant, au niveau génétique ou biochimique, dans la biosynthèse d'un métabolite, c'est à dire des gènes codant pour des polypeptides impliqués dans la voie de biosynthèse d'un métabolite. En particulier, le métabolite peut être une vitamine, un acide aminé, un antibiotique, ou tout autre métabolite bactérien. De préférence, le métabolite est choisi parmi la vitamine B12, la biotine, la riboflavine, la lysine, la thréonine, la méthionine, la pénicilline ou la tétracycline.

Dans un mode préféré de réalisation, les vecteurs selon l'invention contiennent un gène de structure codant pour une protéine donnée, précédé de signaux permettant son expression, et éventuellement la sécretion de ladite protéine.

Un autre objet de l'invention concerne un procédé de production d'une protéine ou d'un métabolite, caractérisé en ce que

- on introduit dans une bactérie gram-négative un vecteur tel que défini ci-dessus contenant le gène de structure codant pour ladite protéine ou un ou plusieurs des gènes intervenant, au niveau génétique ou biochimique, dans la biosynthèse dudit métabolite,
- on cultive cette bactérie dans des conditions d'expression du ou desdits gènes, et
- on récupère la protéine ou le métabolite produits.

Les vecteurs décrits dans la présente invention peuvent être utilisés, à l'echelle industrielle, chez presque toutes les bactéries gram-négatives. Dans le cas du plasmide pXL1635, il peut être utilisé chez toutes les bactéries gram-négatives où le plasmide RK2 se réplique, c'est à dire, toutes les bactéries gram-négatives sauf Myxococcus xanthus, Bradyrhizobium japonicum et les bactéries du genre Bacteroïdes - (Kües et Stahl, 1989). On peut les utiliser de la manière suivante : un ou plusieurs fragments d'ADN portant le ou les gènes qui doivent être, par exemple, amplifiés, sont clonés dans un multisite de clonage par exemple; le plasmide ainsi construit est ensuite introduit dans la bactérie que l'on désire utiliser au niveau industriel. Un système de transformation tel que l'électroporation (Wirth et al, 1989) peut être utilisé pour introduire le plasmide construit. Les clones recombinants sont sélectionnés par la résistance apportée par le plasmide. Ces clones peuvent ensuite être conservés selon les techniques classiques de conservation de souches recombinées pour usage industriel (Standburry et Whitaker, 1984).

L'invention a encore pour objet les bactéries gram-négatives contenant les vecteurs selon l'invention, ainsi que leurs utilisations. En particulier, de telles bactéries peuvent être utilisées pour produire une protéine ou un métabolite, ou directement dans une réaction de bioconversion.

D'autres objets et avantages de la présente invention apparaîtront à la lecture des exemples suivants, qui doivent être considérés comme illustratifs et non limitatifs.

**Techniques générales de clonage, de biologie moléculaire et de biochimie.**

Les méthodes classiques de biologie moléculaire telles que la centrifugation d'ADN plasmidique en gradient de chlorure de césium-bromure d'éthidium, les digestions par des enzymes de restriction, l'électrophorèse sur gel, l'électroélution des fragments d'ADN à partir de gels d'agarose, la transformation dans E. coli, la précipitation des acides nucléiques etc, sont décrites dans la littérature (Maniatis et al.,1982, Ausubel et al., 1987). Les séquences nucléotidiques ont été déterminées par la méthode de terminaison de chaînes en suivant le protocole déjà présenté (Ausubel et al., 1987).

Les enzymes de restriction ont été fournies par New-England Biolabs (Biolabs), Bethesda Reseach Laboratories (BRL) ou Amersham Ltd (Amersham).

Pour les ligatures, les fragments d'ADN sont séparés selon leur taille sur des gels d'agarose à 0,7 % ou d'acrylamide à 8 %, purifiés par électroélution, extraits au phénol, précipités à l'éthanol puis incubés dans un tampon Tris-HCl pH 7.4 50 mM, MgCl$_2$ 10 mM, DTT 10 mM, ATP 2 mM, en présence d'ADN ligase du phage T4 (Biolabs).

Si nécessaire, les fragments d'ADN ayant des extrémités 5' proéminentes sont déphosphorylés par un traitement à la phosphatase alcaline d'intestin de veau (CIP, Pharmacia) à 37°C pendant 30 mn dans le tampon suivant: glycine 100 mM, MgCl$_2$ 1 mM, ZnCl$_2$ 1 mM, pH 10.5. La même technique est utilisée pour la déphosphorylation des extrémités 3' proéminentes ou franches, mais le traitement est de 15 mn à 37°C puis de 15 mn à 56 °C. L'enzyme est inactivée par chauffage du mélange réactionnel à 68°C pendant 15 mn en présence de SDS 1% et de NaCl 100 mM suivi d'une extraction au phénol-chloroforme et d'une précipitation à l'éthanol.

Le remplissage des extrémités 5' proéminentes est effectué par le fragment de Klenow de l'ADN polymérase I d'E. coli (Biolabs). La réaction est effectuée à température ambiante pendant 30 mn dans un tampon Tris-HCl pH 7.2 50 mM, dNTPs 0.4 mM, MgSO$_4$ 10 mM, DTT 0.1 mM, BSA 50 μg/ml. Le remplissage et /ou la digestion des extrémités 3' proéminentes est effectué en présence de l'ADN polymérase du phage T4 (Biolabs) selon les recommandations du fabricant.

Les oligonucléotides sont synthétisés en utilisant la chimie des phosphoramidites protégés en $\beta$ par un groupement cyanoéthyl (Sinha et al., 1984, Giles 1985) avec le synthétiseur automatique d'ADN Biosearch 8600 en utilisant les recommandations du fabricant.

La mutagénèse in vitro par oligodéoxynucléotides est effectuée selon la méthode développée par Taylor et al., 1985, en utilisant le kit distribué par Amersham France, SA.

Les délétions effectuées à l'aide de l'exonucléase III et de la nucléase S1, suivant la technique de Henikoff, 1984, ont été faites en utilisant le kit Erase-a-Base™ System de Promega (Madison, WI, USA) et en suivant le protocole fourni par le fabricant.

Les ADN ligaturés sont utilisés pour transformer la souche rendue compétente: E. coli MC1060 [Δ-(lacIOPZYA)ΦX74, galU, galK, strA$^r$, hsdR](Casadaban et al., 1983) et HB101 [hsdS20, supE44, recA13, ara-14, proA2, lacY1, galK2, rpsL20, xyl-5, mtl-1, λ-] (Maniatis et al., 1982) pour les plasmides ou E. coli TG1 [Δ(lac proA,B), supE, thi, hsdD5/F' traD36, proA$^+$, B$^+$, lacI$^q$, lacZΔM15] (Gibson, 1984) pour les formes réplicatives de phages dérivés du bactériophage M13. La souche polA utilisée est la SF800 (Stachel et al., 1985), cette souche est résistante à l'acide nalidixique (Nal$^r$). Un clone spontanément résistant à la rifampicine (C600 Rif$^r$) (50μg/ml) de la souche C600 [thi-1, thr1, leu-B6, lacY1, tonA21, supE44, λ-] (Maniatis et al, 1982) a été utilisé comme souche réceptrice lors des conjugaisons de E. coli à E. coli.

Les ADN plasmidiques sont purifiés suivant la technique de Birnboim et Doly, 1979. Les minipréparations d'ADN plasmidique sont faites suivant le protocole de Klein et al., 1980.

Le milieu de culture LB est utilisé pour la partie bactériologique (Maniatis et al., 1982). Les souches de Pseudomonas denitrificans SC510 Rif$^r$, Agrobacterium tumefaciens C58C9 (Cameron et al., 1989), Pseudomonas putida KT2440 (Bagdasarian et al., 1981), Rhizobium meliloti 102F34 (Leong et al., 1982) sont aussi cultivées en mileu LB mais la température d'incubation est de 30°C.

Les conjugaisons ont été réalisées comme cela est décrit (Ditta et al., 1980). 10$^9$ cellules de chaque bactérie participant à la conjugaison sont filtrées et adsorbées sur un filtre Millipore de 0.45 μm. Les filtres sont ensuite incubés sur des boites de milieu LB à 37°C puis les cellules sont resuspendues dans 1 ml de 10 mM MgSO$_4$. Des dilutions de la suspension bactérienne sont ensuite étalées sur des boites de mileu LB supplémentées avec des antibiotiques appropriés.

## Exemple 1 : Construction du plasmide pXL1635.

Cet exemple illustre comment un vecteur multihôtes chez les bactéries gram-négatives non mobilisable, et présentant une très grande stabilité ségrégationnelle peut être construit.

## Exemple 1.1 : Obtention d'un dérivé de pRK290 ayant perdu le locus mob.

Cet exemple illustre comment le locus mob de RK2 peut être délété sur un dérivé de RK2.

La délétion de ce locus est rendue difficile en ce sens qu'aucun site unique de restriction n'est connu à proximité du locus mob. Si cela était le cas il aurait été possible de faire une délétion in vitro en utilisant, par exemple l'exonucléase Bal 31 comme cela est décrit (Maniatis et al., 1982). Pour pouvoir réaliser cette délétion, nous avons introduit un site de restriction unique au locus mob de RK2. Ceci a été fait par mutagénèse dirigée selon la technique de Taylor et al, 1985. Les techniques de mutagénèse dirigée ne se font que sur des petits fragments. Nous avons donc procédé en trois étapes: premièrement sous-clonage d'un fragment de RK2 contenant l'origine de transfert (oriT) dans un phage dérivé du phage M13, puis mutagénèse dirigée pour introduire un site de restriction unique; deuxièmement le fragment ainsi muté est ensuite cloné sur le plasmide pUC13 (Viera et Messing, 1982), puis une cassette portant un gène de résistance à la kanamycine est cloné au site de restriction présent dans l'origine de transfert et servira ensuite comme marqueur de la mutation introduite; troisièmement le fragment muté est échangé par double recombinaison avec le fragment sauvage porté par le pRK290. Le plasmide résultant est un dérivé de pRK290 possèdant un site de restriction unique au niveau de l'origine de transfert. Il est donc possible de faire une mutagénèse par délétion en faisant agir des exonucléases puis une DNA ligase. Le détail de ces constructions est donné ci-dessous.

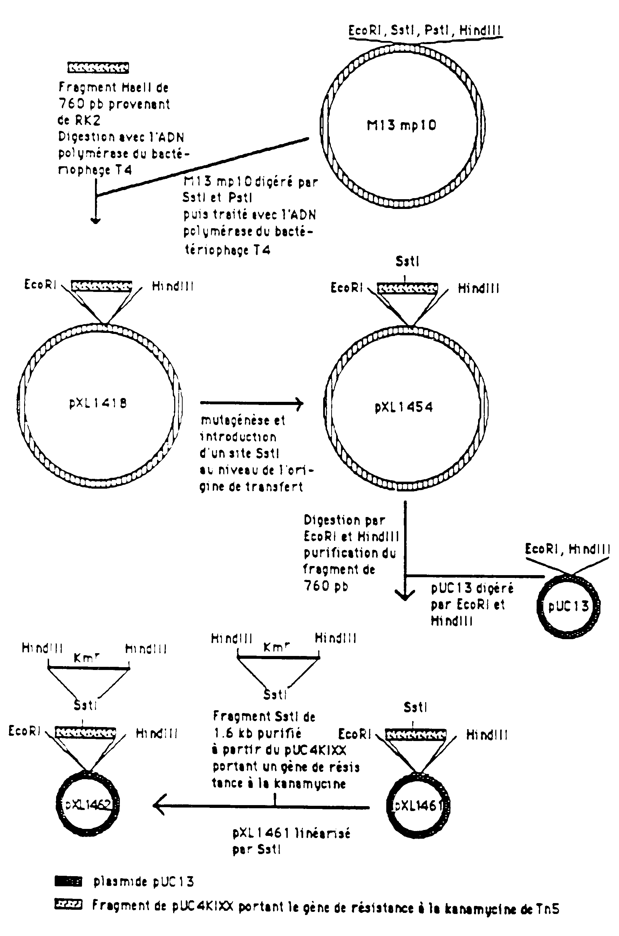

Le fragment HaeII de 760 pb de RK2 qui est suffisant en cis pour obtenir la mobilisation (Guiney et Yakobson, 1982), a été cloné dans M13 mp10 (Viera et Messing, 1982). Ce fragment contient l'origine de transfert de RK2 (Guiney et Yakobson, 1983) et lorsqu'il est cloné sur un plasmide, rend celui-ci mobilisable en trans par les fonctions tra de RK2. Ce fragment a été purifié à partir d'une digestion HaeII de RK2, les extrémités ont été digérées avec l'ADN polymérase du bactériophage T4. Par ailleurs la forme réplicative du phage M13 mp10 a été linéarisée par digestion avec PstI et SstI; les extrémités ont été digérées avec l'ADN polymérase du bactériophage T4. La forme réplicative ainsi obtenue et le fragment purifié ont été ligaturés ensemble puis transformés dans des cellules d'Escherichia coli TG1 rendues compétentes. Les phages recombinants ont été détectés comme cela est décrit (Viera et Messing, 1982). Une forme réplicative résultante a été nommée pXL1418 (voir figure 1).

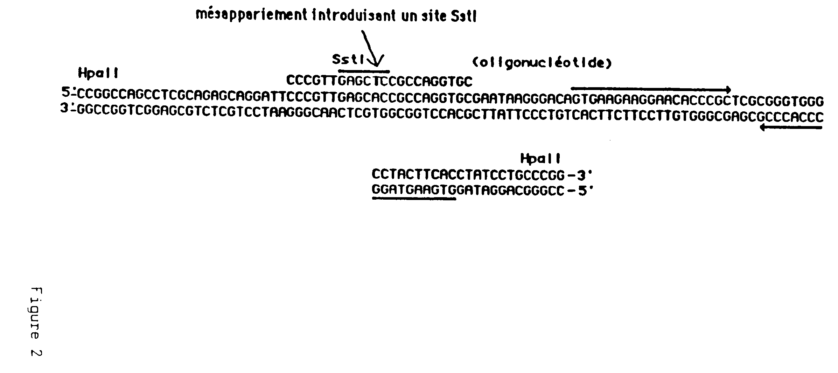

La séquence du fragment HpaII de 112 pb contenant l'origine de transfert de RK2 a été publiée (Guiney et Yakobson, 1983). Ce fragment est contenu dans le fragment HaeII de 760 pb porté dans le pXL1418. Il a été observé qu'il existe une séquence inversée répétée capable de former une structure en épingle à cheveux, avec 19 pb pour chaque séquence répétée (voir figure 2). Une mutagénèse dirigée selon la méthode développée par Taylor et al. (1985), a été réalisée sur l'ADN phagique simble brin obtenu à partir de cette forme réplicative. L'oligonucléotide utilisé a la séquence suivante : 5'-CCC GTT GAG CTC CGC CAG GTG C-3'. Sur la figure 2 sont portées la séquence du fragment HpaII de 112 pb ainsi que celle de l'oligonucléotide; comme il est indiqué sur cette figure la séquence de l'oligonucléotide ne diffère de celle du fragment que par un seul nucléotide; cette mutation introduit un site SstI. Il a d'abord été vérifié que l'oligonucléotide était bien complémentaire de l'ADN simple brin obtenu par infection de E. coli TG1 avec la forme réplicative du pXL1418. Pour ce faire l'oligonucléotide en question a été utilisé comme amorce de séquence nucléotidique. Il a bien été observé qu'une réaction de séquence avait eu lieu. La séquence nucléotidique est lue et les premières bases correspondent bien à la séquence de l'extrémité droite du brin supérieur présenté à la figure 2.

La mutagénèse dirigée a été réalisée comme cela est décrit ci-dessus. Des clones mutants sont obtenus présentant bien un site SstI introduit à la position attendue. Une de ces formes réplicatives a été nommée pXL1454 (voir figure 1).

Le pXL1454 est digéré par EcoRI et HindIII. Le fragment de 760 pb est purifié, puis mis à ligaturer avec du pUC13 digéré par EcoRI et HindIII. Après transformation, des clones recombinants ont été obtenus. Un clone plasmidique a été retenu et nommé pXL1461 (voir figure 1).

L'étape suivante a consisté à introduire une cassette conférant la résistance à un antibiotique au site SstI afin qu'un marqueur sélectif soit présent dans celui-ci. Pour ce faire, le pXL1461 a été linéarisé par SstI puis ligaturé avec le fragment SstI purifié du pUC4KIXX (Pharmacia France, SA). Ce fragment de 1.6 kb porte le gène de résistance à la kanamycine du transposon Tn5. Les clones recombinants ont été sélectionnés après transformation sur milieu LB supplémenté avec de l'ampicilline et de la kanamycine. Le plasmide ainsi obtenu a été nommé pXL1462 (voir figure 1).

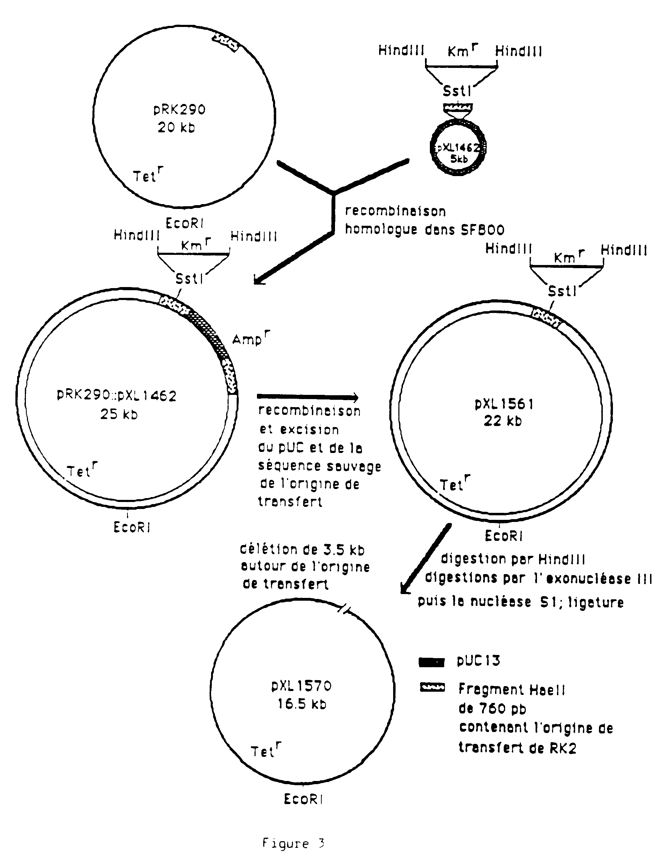

Le plasmide pRK290 est transformé dans une souche de Escherichia coli polA, SF800 (Stachel et al., 1985). Dans une telle souche les plasmides dérivés de RK2 se répliquent car leur réplication est indépendante de l'ADN polymérase I de E. coli tandis que pUC13 ne se réplique pas. Le pXL1462 est ensuite transformé dans la souche SF800 pRK290. Après transformation, les bactéries sont étalées sur milieu LB supplémenté avec de la tétracycline, de la kanamycine et de l'ampicilline. Etant donné que le plasmide pXL1462 ne peut se répliquer chez cette souche, l'acquisition des résistances portées par ce plasmide ne peut se faire que par recombinaison homologue entre les deux plasmides. En effet ces deux plasmides ont en commun le fragment HaeII de 760 pb portant l'origine de transfert. Il peut donc y avoir recombinaison homologue entre les régions homologues des deux plasmides ce qui résulte en une fusion entre les deux réplicons. L'ADN plasmidique d'un clone résistant aux trois antibiotiques a été analysé par restriction. Il s'avère par analyse sur gel d'agarose qu'il ne contient qu'un seul plasmide. L'analyse de ce plasmide par digestions de restriction montre que le pXL1462 a bien recombiné avec le plasmide pRK290 au niveau de l'origine de transfert. Le plasmide résultant de la fusion des deux réplicons porte deux origines de transfert de RK2 : une sauvage et une possèdant un site SstI introduit par mutagénèse dans lequel se trouve clonée une cassette portant un gène de résistance à la kanamycine (voir figure 3). Un clone

7

contenant un tel plasmide est ensuite cultivé par cultures dilutions en milieu LB supplémenté avec de la tétracycline et de la kanamycine. L'absence d'ampicilline permettra que les événements de recombinaison simple avec excision du pUC13 portant la copie sauvage du fragment de 760 pb de l'origine de transfert ne soient pas contre sélectionnés. Comme le pUC13 et les plasmides dérivés ne se répliquent pas, le plasmide excisé sera perdu tout de suite. Après une série de cultures dilutions, représentant 60 générations, en milieu liquide sélectif pour le pRK290 et pour le marqueur de l'origine de réplication mutée, les bactéries sont étalées sur milieu LB supplémenté avec les mêmes antibiotiques. 2000 colonies sont repiquées sur le même milieu et sur des boîtes de milieu LB supplémentées avec de l'ampicilline. 2 clones sensibles à l'ampicilline sont obtenus. Les plasmides portés par ces clones sont purifiés et analysés par restriction. Ces analyses montrent que ces plasmides correspondent exactement à l'excision du pUC13 avec la copie sauvage de l'origine de transfert de RK2. Ce plasmide est nommé pXL1561 (voir figure 3). Comme ce plasmide possède deux sites HindIII au niveau de l'origine de transfert, il est possible de faire une délétion par digestion par une exonucléase après digestion du plasmide par HindIII (voir figure 3). Le plasmide pXL1561 a donc été digéré par HindIII puis une série de délétions ont été réalisées par la technique de Henikoff (1984); cette technique utilise l'action successive de l'exonucléase III et de la nucléase S1; les délétions obtenues sont bidirectionnelles. Ces délétions sont ensuite mises à ligaturer, et la ligature est transformée dans MC1060. L'ADN plasmidique de transformants ainsi obtenus est analysé par digestions de restriction. Plusieurs clones montrent avoir subi une délétion au niveau du locus mob. Nous avons retenu un plasmide qui avait subi une délétion de 3.5 kb. Cette délétion doit correspondre, si l'exonucléase III a digéré l'ADN du plasmide avec les mêmes cinétiques de part et d'autre des sites HindIII, à une délétion de 1,75 kb de part et d'autre de l'origine de transfert du plasmide. Le plasmide ainsi obtenu est nommé pXL1570 (voir figure 3).

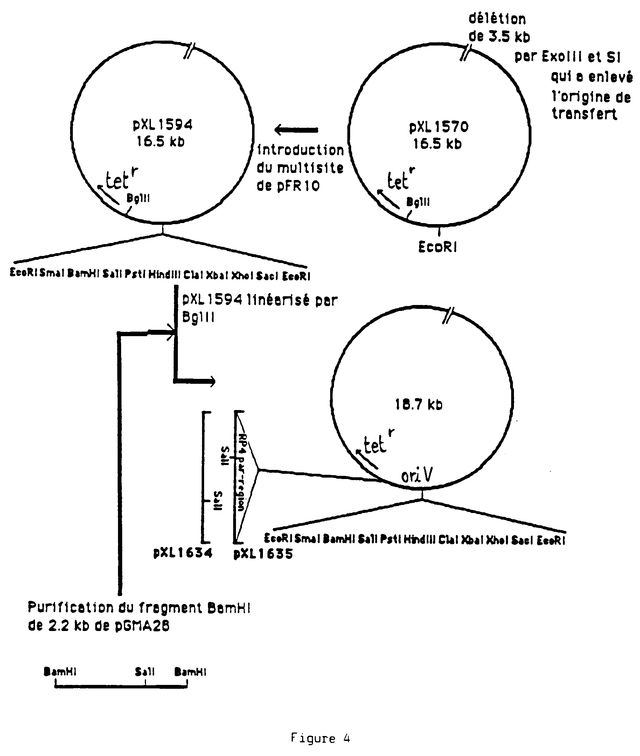

Le pXL1570 possède EcoRI et BglII comme sites uniques de restriction. Nous avons recherché à introduire un multisite de clonage au site EcoRI afin que le plasmide résultant contienne plusieurs sites de clonage. Le plasmide pFR10 (Shapira et al., 1983) qui possède un multisite de clonage bordé par deux sites EcoRI est utilisé comme source d'un multisite de clonage. Le pFR10 est digéré par EcoRI, tout comme le pXL1570. Les deux digestions sont mises à ligaturer et la ligature est transformée dans E. coli MC1060. Des clones recombinants sont obtenus, dont un, nommé pXL1594 (voir figure 4), dont l'orientation du multisite est déterminée.

## Exemple 1.2 : Construction d'un dérivé de pRK290 non mobilisable et possédant le locus par de RK2.

Cet exemple illustre comment en clonant un fragment portant le locus par de RP4 sur un dérivé de RK2 on obtient un plasmide qui est plus stable ségrégationnellement.

Le fragment BamHI de 2,2 kb du pGMA28 (Gerlitz et al., 1988) contenant le locus par de RP4 est purifié puis mis à ligaturer avec pXL1594 linéarisé par BglII. La ligature est transformée dans E. coli MC1060. L'ADN plasmidique de 24 transformants est analysé par digestion de restriction, et 3 clones sont porteurs du fragment de 2,2 kb. 2 d'entre eux possèdent le fragment dans la même orientation, le troisième correspondant à l'orientation inverse. Le plasmide correspondant aux deux premiers clones est nommé pXL1635 tandis que celui contenant le fragment dans l'autre orientation est appelé pXL1634 (voir figure 4).

Les plasmides ainsi obtenus sont des dérivés de RK2, et plus précisément encore, de pRK290, avec une délétion de 3,5 kb autour de l'origine de transfert, l'intégration de la région par de RP4 et, optionnellement, d'un multisite de clonage. Les sites suivants peuvent être utilisés pour des clonages symétriques: EcoRI, BamHI, PstI, HindIII, ClaI,XbaI, XhoI et SstI; pour des clonages asymétriques EcoRI doit être exclu mais les autres enzymes peuvent être utilisées.

## Exemple 2 : Mobilisation de pXL1635.

La mobilisation du pXL1635 a été étudiée. Celle-ci est effectuée sur un transfert conjugatif de E. coli à E. coli. Un transfert à trois partenaires (selon la technique dèjà décrite de Ditta et al., 1980) a été effectué en utilisant les souches suivantes

- souche portant le plasmide à mobiliser: HB101 (pXL1635) ou HB101 (pRK290) ou HB101 (pXL1570) ou HB101 (pUC9tet).
- souche contenant le plasmide "helper" pour la mobilisation des dérivés de RK2: HB101 pRK2013
- souche réceptrice: C600 Rif$^r$.

Le plasmide pUC9tet provient de chez Pharmacia France S. A.; il s'agit d'un plasmide non mobilisable, dérivé de pBR322 conférant la même résistance que les plasmides pRK290, pXL1635 et pXL1570.

Les transconjugants sont sélectionnés sur milieu LB supplémenté avec de la tétracycline et de la rifampicine. Les fréquences de conjugaison sont calculées comme cela est habituellement effectué (Ditta et al., 1980) comme étant le ratio du nombre de cellules réceptrices ayant effectivement acquis la résistance du plasmide sur le nombre total de cellules réceptrices. La conjugaison est effectuée avec HB101 comme souche donatrice. Il s'agit d'une souche recA, ce qui évite la recombinaison homologue qui pourrait se produire entre le pRK2013 et pXL1570, pRK290, pXL1635 et pUC9tet puisque ces plasmides ont des séquences homologues.

**Tableau: Fréquence de conjugaison de dérivés de RK2 de E. coli HB101 vers C600 Rif$^r$. Les détails de l'expérience sont donnés dans le texte; - : absence de plasmide donneur.**

| Plasmide | Fréquence de conjugaison |
|----------|--------------------------|
| pXL1635 | $<10^{-5}$ |
| pXL1570 | $<10^{-5}$ |
| pRK290 | 0,2 |
| - | $<10^{-8}$ |
| pUC9tet | $<10^{-5}$ |

Comme il ressort clairement de ce tableau, la perte du locus mob se traduit par une perte presque totale de la mobilisation (il y a au moins un facteur $2.10^4$ ) entre la fréquence de conjugaison du pXL1570 et celle du pRK290 alors que ces plasmides ne différent que par la délétion de 3,5 kb). Le pXL1635 présente les mêmes propriétés de mobilisation que le pXL1570. Il est probable, comme cela a déjà été décrit précédemment, que la conjugaison observée ici avec le pXL1570 et le pXL1635 est due à une recombinaison homologue entre ces plasmides (qui dérivent directement de pRK290) et le pRK2013 qui ont des régions d'homologies (Ditta et al, 1980). Le plasmide contrôle, non mobilisable, pUC9tet est mobilisé à des fréquences qui ne sont pas différentes de celles observées pour le pXL1635 et le pXL1570 ce qui montre que ces plasmides ont bien perdu leur propriété de mobilisation et qu'ils ne peuvent être mobilisés que par recombinaison, même si dans l'expérience décrite les souches donneuses sont recA (il doit, dans cette expérience s'agir de recombinaison recA indépendante). Le mécanisme de transfert conjugatif de pRK290 étant le même entre deux souches de E. coli et entre deux bactéries Gram-négatives, le plasmide pXL1635 doit être considéré comme non mobilisable quel que soit l'hôte.

**Exemple 3 : Introduction du pXL1635 chez des bactéries gram-négatives autres qu'Escherichia coli.**

Cet exemple illustre comment le plasmide, non mobilisable, pXL1635 peut être introduit chez diverses bactéries gram-négatives.

Comme le plasmide pXL1635 ne porte pas d'origine de transfert, il ne peut être mobilisé de Escherichia coli vers une autre bactérie gram-négative. Pour l'introduction du pXL1635 chez diverses bactéries gram-négatives la méthode de transformation par électroporation (Wirth et al., 1989) est utilisée. Les bactéries suivantes sont transformées, avec les plasmides pRK290, pXL1570 et pXL1635 par cette méthode:
- Pseudomonas denitrificans SC510 Rif$^R$
- Agrobacterium tumefaciens C58C9
- Pseudomonas putida KT2440
- Rhizobium meliloti 102F34

Pour chaque bactérie, un clone recombinant est réisolé; l'ADN plasmidique est purifié et s'avère ne pas être différent de celui du plasmide de départ par plusieurs digestions de restriction. Le plasmide pXL1635 peut donc être introduit chez les bactéries gram-négatives.

**Exemple 4 : Stabilité de pXL1635**

Cet exemple illustre comment le pXL1635 est plus stable que le pRK290 chez diverses bactéries gram-négatives.

Les différentes bactéries recombinantes ainsi que les souches parentes sont cultivées en milieu LB sans antibiotique additionné. Lorque la phase stationnaire est atteinte, une dilution au 1/1000 est réalisée. Ces souches sont ainsi cultivées en moyenne pendant 50 générations (le nombre de générations est estimé à 50 et est compris dans une fourchette de 40 à 60). Des dilutions des cultures sont ensuite étalées sur milieu LB gélosé. Pour chaque culture, 200 clones indépendants sont repiqués sur milieu sélectif pour le plasmide. La fréquence des clones ayant perdu la résistance, et donc le plasmide, a été calculée. Cette valeur est portée sur le tableau ci-dessous.

Tableau

| Fréquences, en%, de perte des plasmides pRK290, pXL1570 et pXL1635 chez diverses bactéries gram-négatives. | | | |
|---|---|---|---|
| Souches | pRK290 | pXL1570 | pXL1635 |
| -Escherichia coli MC1060 | 2 | 3 | 0 |
| -Pseudomonas denitrificans SC510Rif$^R$ | 15 | 13 | 1 |
| -Agrobacterium tumefaciens C58C9 | 25 | 27 | 0 |
| -Pseudomonas putida KT2440 | 1 | 2 | 0 |
| -Rhizobium meliloti 102F34 | 2 | 2.5 | 0 |

Les valeurs correspondent à la fréquence, en %, de clones ayant perdu la résistance du plasmide après 50 générations de culture, non sélectives, en milieu LB.

De ce tableau, il ressort clairement que le pXL1635 est beaucoup plus stable que le pRK290 et que le pXL1570; cette propriété est vraie chez toutes les bactéries étudiées. Ces deux derniers plasmides ont les mêmes fréquences de clones ayant perdu le plasmide; les différences observées ne sont pas significatives du point de vue statistique. La délétion de 3,5 kb n'affecte donc pas les propriétés de réplication et de stabilité du pRK290. Par contre, le clonage du fragment de 2,2 kb du pGMA28 entraîne un gain de stabilité très important. Ces résultats montrent clairement que le plasmide pXL1635 est un plasmide qui présente des propriétés beaucoup plus intéressantes que le pRK290 du point de vue de la stabilité. Ces avantages sont bien entendu les mêmes sur les autres dérivés de RK2 qui sont, d'après ce qui est publié, moins avantageux que pRK290 pour une utilisation industrielle (Schmidhauser et al., 1988).

**Description des figures**

Figure 1: Construction des pXL1418, pXL1454, pXL1461 et pXL1462. Le détail des constructions est donné dans le texte.

Figure 2 : Séquence du fragment HpaII de 110 pb contenant l'origine de transfert de RK2 (d'après Guiney et Yakobson, 1983). La séquence de l'oligonucléotide qui a servi pour la mutagénèse est indiquée ainsi que le site SstI qui est introduit. Les deux séquences inversées répétées qui doivent former une structure en épingle à cheveux sont soulignées par une flèche.

Figure 3: Construction des pXL1561 et pXL1570. Le détail des constructions est donné dans le texte.

Figure 4: Construction des pXL1594, pXL1635 et pXL1634. Le détail des constructions est donné dans le texte.

**Définition des termes employés et abréviations.**

ADN recombinant: ensemble de techniques qui permettent soit d'associer au sein du même microorganisme des séquences d'ADN qui ne le sont pas naturellement, soit de mutagéniser spécifiquement un fragment d'ADN.

ATP: adénosine 5'-triphosphate

BSA: sérum albumine bovine

Codon stop: codon de terminaison de traduction

dNTP: 2'-désoxyribonucléosides 5'-triphosphates

DTT: dithiothréitol

kb: kilobases

pb: paires de bases

## Références bibliographiques

Ausubel F. M., R. Brent, R. E. Kinston, D. D. Moore, J. A. Smith, J. G. Seidman and K. Struhl. 1987. Current protocols in molecular biology 1987-1988. John Willey and Sons, New York.

Bagdasarian, J. Frey, and K. Timmis. 1981. Specific-purpose plasmid cloning vectors. II. Broad host range, high copy number, RSF1010-derived vectors, and a host vector system for gene cloning in Pseudomonas. Gene 16, 237-247.

Cameron B, K. Briggs, S. Pridmore, G. Brefort and J. Crouzet, 1989. Cloning and analysis of genes involved in coenzyme B12 biosynthesis in Pseudomonas denitrificans . J. Bacteriol, 171, 547-557.

Casadaban, M. J., A. Martinez-Arias, S. T. Shapira and J. Chou. 1983. $\beta$-galactosidase gene fusion for analysing gene expression in Escherichia coli and Yeast. Methods Enzymol. 100, 293-308.

Derbyshire, K. M. and N. S. Willets. 1987. Mobilization of the non-conjugative plasmid RSF1010: a genetic analysis of its origin of transfer. Mol. Gen. Genet., 206, 154-160.

Ditta G., Schmidhauser T., Yakobson E., Lu P., Liang X.-W., Finlay D. R., Guiney D., Helinski D. R., 1985. Plasmids related to the broad host range vector pRK290, useful for gene cloning and for monitoring gene expression. Plasmid, 13, 149-154.

Ditta G., Stanfield S., Corbin D., Helinski D. R., 1980. Broad host range DNA cloning system for Gram-negative bacteria: Construction of a gene bank of Rhizobium melitoti. Proc. Natl. Acad. Sci. U. S. A., 77, 7347-7351.

Gerlitz, M., O. Hrabak and H. Schwab. Abstract 8th International Biothechnology Symposium, 1988, A156, p. 129.

Giles, J. W. 1985. Adances in automated DNA synthesis. Am. Biotechnol., Nov./Dec.

Guidelines for research involving recombinant DNA molecules; Notice. National Institute of Health, Federal Register, Vol. 51, No. 88, 16958-16965.

Guiney, D. G. and E. Yakobson. 1983. Location and nucleotide sequence of the transfer origin of the broad host range plasmid RK2. Proc. Natl. Acad. Sci. USA, 80, 3595-3598.

Henikoff S. 1984. Unidirectional digestion with exonuclease III creates targeted breakpoints for DNA sequencing. Gene, 28, 351-359.

Keen, N. T., S. Tamaki, D. Kobayashi and D. Trollinger. 1988. Improved broad host range plasmids for DNA cloning in gram-negative bacteria. Gene, 70, 191-197.

Kües, U. and U. Stahl. 1989. Replication of plasmid in gram-negative bacteria. Mic. Rev., 53, 491-516.

Leong, L. S., G. S. Ditta, and D. R. Helinski. 1982. Identification of a cloned gene coding for $\delta$-aminolevulinic acid synthetase from Rhizobium melitoti. J. Biol. Chem. 257, 8724-8730.

Maniatis, T., E. F. Fritsch, and J. Sambrook. 1982. Molecular cloning: a laboratory manual. Cold Spring Harbor Laboratory, Cold Spring Harbor, New York.

Saurugger, P. N., O. Hrabak, H. Schwab and R. M. Lafferty. 1986. Mapping and cloning of the par-region of broad-host-range plasmid RP4. J. Biotechnol., 4, 333-343.

Schmidhauser, T. J. and D. R. Helinski. 1985. Regions of broad-host-range plasmid RK2 involved in replication and stable maintenance in nine species of gram-negative bacteria. J. Bacteriol., 164, 446-455.

Schmidhauser, T. J., G. Ditta and D. R. Helinski. 1988. Broad-Host-Range plasmid cloning vectors for gram-negative bacteria. In R. L. Rodriguer and D. T. Denhardt (ed.), Vectors, a survey of molecular cloning vectors and their uses, Butterworths, Boston.

Scholz, P., V. Haring, B. Wittman-Liebholt, K. Ashman, M. Bagdasarian and E. Scherzinger. 1989. Complete nucleotide sequence and gene organization of the broad-host-range plasmid RSF1010. Gene, 75, 271-288.

Shapira S. K., J. Chou , F. V. Richaud, and M. C. Casadaban. 1983. New versatile plasmid vector for expresssion of hybrid proteins coded by a cloned gene fused to lacZ gene sequences encoding enzymatically active carboxy-terminal portion of $\beta$-galactosidase. Gene 25, 71-83.

Sinha, N. D., J. Biernat, J. McManus and H. Köster. 1984. Polymer support oligonucleotide synthesis, XVIII: Use of $\beta$-cyanoethyl-N,N-dialkylamino-/N-morpholino phosphoramidite of deoxynucleosides for the synthesis of DNA fragments simplifying deprotection and isolation of the final product. Nucl. Acids Res., 12, 4539-4557.

Stachel S. E., G. An, C. Flores and E. W. Nester. 1985. A Tn3lacZ transposon for the random generation of $\beta$-galactosidase gene fusions: application to the analysis of gene expression in Agrobacterium. Embo J., 4, 891-898.

Stanburry, P. F. and A. Whitaker. 1984. Principles of fermentation technology. Pergamon Press, Oxford.

Taylor J. W., J. Ott and F. Eckstein. 1985. The rapid generation of oligonucleotide-directed mutations at high frequency using phophorothioate-modified DNA. Nucl. Acid Res., 13, 8764-8765.

Thomas C.M. 1986. Evidence for the involvement of the incC locus of broad host range plasmid RK2 in plasmid maintenance. Plasmid, 16, 15-29.

Trevors, J. T., T. Barkay and A. W. Bourquin. 1987. Gene transfer among bacteria in soil and aquatic environments: a review. Can. J. Microbiol., 33, 191-198.

Viera J. and J. Messing. 1982. The pUC plasmids, an M13mp7-derived system for insertion mutagenesis and sequencing with synthetic universal primers. Gene, 19, 259-268.

Wirth, R., A. Frieseneger and S. F. Fielder. 1989. Transformation of variu-ous species of gram-negative bacteria belonging to 11 different genera by electroporation. Mol. Gen. Genet., 216, 175-177.

**Revendications**

1. Vecteur de clonage et/ou d'expression à large spectre d'hôtes caractérisé en ce qu'il possède une origine de réplication dérivée d'un plasmide à large spectre d'hôtes chez les bactéries gram-négatives du groupe d'incompatibilité P, lui permettant de se répliquer chez presque toutes les bactéries gram-négatives, contient un fragment d'ADN portant la région par du plasmide RP4, et est non mobilisable.

2. Vecteur selon la revendication 1 caractérisé en ce qu'il possède l'origine de réplication du plasmide RK2.

3. Vecteur selon la revendication 1 dans lequel le locus mob a été délété.

4. Vecteur selon la revendication 1 caractérisé en ce qu'il contient en outre un multisite de clonage.

5. Vecteur selon la revendication 1 caractérisé en ce qu'il contient en outre un marqueur de sélection, constitué de préférence par un gène de résistance à un antibiotique.

6. Vecteur de clonage et/ou d'expression à large spectre d'hôtes chez les bactéries gram-négatives caractérisé en ce qu'il dérive du plasmide RK2, a une taille inférieure à 20 kb, contient un fragment d'ADN portant la région par du plasmide RP4, ne contient pas de locus mob, et éventuellement, contient un multisite de clonage et un marqueur de sélection.

7. Le plasmide pXL1635 caractérisé en ce qu'il dérive du plasmide RK2, a une taille inférieure à 20 kb, contient un fragment d'ADN portant la région par du plasmide RP4, ne contient pas de locus mob, et contient un multisite de clonage et un gène de résistance à un antibiotique., tel que représenté sur la figure 4.

8. Vecteur selon l'une des revendications précédentes caractérisé en ce qu'il contient en outre un ADN recombinant.

9. Vecteur selon la revendication 8 ccaractérisé en ce que l'ADN recombinant contient, sous la dépendance de séquences de régulation de l'expression génétique, un ou plusieurs gènes de structure codant pour un ou plusieurs polypeptides donnés.

10. Vecteur selon la revendication 9 caractérisé en ce que les gènes de structure peuvent être sous la dépendance de leurs propres séquences de régulation, ou de séquences différentes.

11. Vecteur selon l'une des revendications 9 ou 10 caractérisé en ce que les séquences de régulation de l'expression génétique sont constituées par un ou plusieurs élements choisis parmi les promoteurs, les terminateurs, les peptides signaux, et les sites de fixation des ribosomes.

12. Vecteur selon la revendication 9 caractérisé en ce que le ou les gènes de structure codent pour une protéine, qui peut être choisie parmi les enzymes, les hormones, et les facteurs de croissance, et de préférence, parmi l'albumine, le tPA, le TIMP, les interleukines, les apolipoprotéines, et les interférons.

**13.** Vecteur selon la revendication 9 caractérisé en ce que le ou les gènes de structure sont des gènes intervenant, au niveau génétique ou biochimique, dans la biosynthèse d'un métabolite.

**14.** Vecteur selon la revendication 13 caractérisé en ce que le métabolite est choisi parmi les vitamines, les antibiotiques, les acides aminés et tout autre métabolite bactérien.

**15.** Vecteur selon la revendication 14 caractérisé en ce que le métabolite est choisi parmi la vitamine B12, la biotine, la riboflavine, la lysine, la thréonine, la méthionine, la pénicilline, et la tétracycline.

**16.** Procédé de production d'une protéine ou d'un métabolite, caractérisé en ce que
- on introduit dans une bactérie gram-négative un vecteur selon l'une des revendications 8 à 15 contenant le gène de structure codant pour ladite protéine ou un ou plusieurs des gènes intervenant, au niveau génétique ou biochimique, dans la biosynthèse dudit métabolite,
- on cultive cette bactérie dans des conditions d'expression du ou desdits gènes, et
- on récupère la protéine ou le métabolite produits.

**17.** Bactérie gram-négative contenant un vecteur selon l'une des revendications 1 à 15.

**18.** Utilisation d'une bactérie selon la revendication 17 pour produire une protéine ou un métabolite.

**19.** Utilisation d'une bactérie selon la revendication 17 directement dans une réaction de bioconversion.

**Claims**

1. Cloning and/or expression vector with a broad host range characterized in that it has a replication origin derived from a plasmid with a broad host range in Gram-negative bacteria of the incompatibility group P, allowing it to replicate in practically all Gram-negative bacteria, has a DNA fragment carrying the par region of the plasmid RP4, and is non-mobilizable.

2. Vector according to Claim 1, characterized in that it has the replication origin of the plasmid RK2.

3. Vector according to Claim 1, from which the mob locus has been deleted.

4. Vector according to Claim 1, characterized in that it contains, in addition, a multiple cloning site.

5. Vector according to Claim 1, characterized in that it contains, in addition, a selectable marker, preferably consisting of a gene for resistance to an antibiotic.

6. Cloning and/or expression vector with a broad host range in Gram-negative bacteria, characterized in that it is derived from the plasmid RK2, has a size of less than 20 kb, contains a DNA fragment carrying the par region of the plasmid RP4, does not contain the mob locus, and optionally, contains a multiple cloning site and a selectable marker.

7. Plasmid pXL1635, characterized in that it is derived from the plasmid RK2, has a size of less than 20 kb, contains a DNA fragment carrying the par region of the plasmid RP4, does not contain the mob locus, and contains a multiple cloning site and a gene for resistance to an antibiotic, as represented in Figure 4.

8. Vector according to one of the preceding claims, characterized in that it contains, in addition, a recombinant DNA.

9. Vector according to Claim 8, characterized in that the recombinant DNA contains, in dependence on sequences for regulation of genetic expression, one or more structural genes encoding one or more given polypeptides.

10. Vector according to Claim 9, characterized in that the structural genes may be in dependence on their own regulatory sequences, or on different sequences.

**11.** Vector according to one of Claims 9 or 10, characterized in that the sequences for regulation of genetic expression consist of one or more elements chosen from promoters, terminators, signal peptides, and ribosome-binding sites.

**12.** Vector according to Claim 9, characterized in that the structural genes encode a protein, which may be chosen from enzymes, hormones, growth factors, and preferably from albumin, tPA, TIMP, interleukins, apolipoproteins, and interferons.

**13.** Vector according to Claim 9, characterized in that the structural gene(s) are genes involved, at the genetic or biochemical level, in the biosynthesis of a metabolite.

**14.** Vector according to Claim 13, characterized in that the metabolite is chosen from vitamins, antibiotics, amino acids and any other bacterial metabolite.

**15.** Vector according to Claim 14, characterized in that the metabolite is chosen from vitamin B12, biotin, riboflavine, lysine, threonine, methionine, penicillin, and tetracycline.

**16.** Process for the production of a protein or a metabolite, characterized in that
- a vector according to one of Claims 8 to 15 containing the structural gene encoding the said protein or one or more genes involved, at the genetic or biochemical level, in the biosynthesis of the said metabolite, is introduced into a Gram-negative bacterium,
- this bacterium is cultured under conditions for expression of the said gene(s), and
- the protein or the metabolite produced are recovered.

**17.** Gram-negative bacterium containing a vector according to one of Claims 1 to 15.

**18.** Use of a bacterium according to Claim 17 to produce a protein or a metabolite.

**19.** Use of a bacterium according to Claim 17 directly in a bioconversion reaction.

**Patentansprüche**

**1.** Clonierungs- und/oder Expressionsvektor mit einem breiten Wirtsspektrum, dadurch **gekennzeichnet**, daß er einen Replikationsorigin, abgeleitet von einem Plasmid mit einem breiten Wirtsspektrum bei den gramnegativen Bakterien der Inkompatibilitätsgruppe P, besitzt, der ihm die Replikation in fast allen gramnegativen Bakterien erlaubt, ein DNA-Fragment, das die par-Region des Plasmids RP4 trägt, enthält und nicht mobilisierbar ist.

**2.** Vektor nach Anspruch 1, dadurch **gekennzeichnet**, daß er den Replikationsorigin des Plasmids RK2 besitzt.

**3.** Vektor nach Anspruch 1, worin der mob-Locus deletiert worden ist.

**4.** Vektor nach Anspruch 1, dadurch **gekennzeichnet**, daß er weiterhin eine Mehrfachclonierungsstelle enthält.

**5.** Vektor nach Anspruch 1, dadurch **gekennzeichnet**, daß er weiterhin einen Selektionsmarker, bestehend bevorzugt aus einem Antibiotika-Resistenzgen, enthält.

**6.** Clonierungs- und/oder Expressionsvektor mit einem breiten Wirtsspektrum bei gramnegativen Bakterien, dadurch **gekennzeichnet**, daß er von dem Plasmid RK2 abgeleitet ist, kleiner als 20 kb ist, ein DNA-Fragment, das die par-Region des Plasmids RP4 trägt, enthält, keinen mob-Locus enthält und gegebenenfalls eine Mehrfachclonierungsstelle und einen Selektionsmarker enthält.

**7.** Plasmid pXL1635, dadurch **gekennzeichnet**, daß es von dem Plasmid RK2 abgeleitet ist, kleiner als 20 kb ist, ein DNA-Fragment, das die par-Region des Plasmids RP4 trägt, enthält, keinen mob-Locus enthält und eine Mehrfachclonierungsstelle und ein Antibiotika-Resistenzgen enthält, wie in Figur 4 dargestellt.

8. Vektor nach einem der vorausgehenden Ansprüche, dadurch **gekennzeichnet**, daß er weiterhin eine rekombinante DNA enthält.

9. Vektor nach Anspruch 8, dadurch **gekennzeichnet**, daß die rekombinante DNA in Abhängigkeit von Sequenzen zur Regulation der genetischen Expression ein oder mehrere Strukturgene, die ein oder mehrere gegebene Polypeptide codieren, enthält.

10. Vektor nach Anspruch 9, dadurch **gekennzeichnet**, daß die Strukturgene unter der Abhängigkeit ihrer eigenen Regulationssequenzen oder verschiedener Sequenzen stehen können.

11. Vektor nach einem der Ansprüche 9 oder 10, dadurch **gekennzeichnet**, daß die Sequenzen zur Regulation der genetischen Expression aus einem oder mehreren Elementen, ausgewählt aus Promotoren, Terminatoren, Signalpeptiden und Ribosomen-Bindungsstellen bestehen.

12. Vektor nach Anspruch 9, dadurch **gekennzeichnet**, daß das oder die Strukturgen(e) ein Protein codiert/codieren, das aus Enzymen, Hormonen, Wachstumsfaktoren und bevorzugt aus Albumin, tPA, TIMP, Interleukinen, Apolipoproteinen und Interferonen ausgewählt ist.

13. Vektor nach Anspruch 9, dadurch **gekennzeichnet**, daß das oder die Strukturgen(e) Gene ist/sind, die auf genetischer oder biochemischer Ebene an der Biosynthese eines Metaboliten beteiligt sind.

14. Vektor nach Anspruch 13, dadurch **gekennzeichnet**, daß der Metabolit aus Vitaminen, Antibiotika, Aminosäuren und jedem anderen bakteriellen Metaboliten ausgewählt ist.

15. Vektor nach Anspruch 14, dadurch **gekennzeichnet**, daß der Metabolit aus Vitamin $B_{12}$, Biotin, Riboflavin, Lysin, Threonin, Methionin, Penicillin und Tetracyclin ausgewählt ist.

16. Verfahren zur Herstellung eines Proteins oder eines Metaboliten, dadurch **gekennzeichnet**, daß man
    - in ein gramnegatives Bakterium einen Vektor nach einem der Ansprüche 8 bis 15, der das Strukturgen, das das Protein codiert, oder ein oder mehrere Gene, die auf genetischer oder biochemischer Ebene an der Biosynthese des Metaboliten beteiligt sind, enthält, einschleust,
    - dieses Bakterium unter Expressionsbedingungen des oder der Gens/Gene züchtet, und
    - das gebildete Protein oder den gebildeten Metaboliten isoliert.

17. Gramnegatives Bakterium, enthaltend einen Vektor nach einem der Ansprüche 1 bis 15.

18. Verwendung eines Bakteriums nach Anspruch 17 zur Herstellung eines Proteins oder eines Metaboliten.

19. Verwendung eines Bakteriums nach Anspruch 17 direkt in einer Biokonversionsreaktion.

Figure 1

mésappariement introduisant un site SstI

SstI ↓   (oligonucléotide)

CCCGTTGAGCTCCGCCAGGTGC

Hpall
5'-CCGGCCAGCCTCGCAGAGCAGGATTCCCGTTGAGCACCGCCAGGTGCGAATAAGGGACAGTGAAGAAGGAACACCCGCTCGCGGGTGGG
3'-GGCCGGTCGGAGCGTCTCGTCCTAAGGGCAACTCGTGGCGGTCCACGCTTATTCCCTGTCACTTCTTCCTTGTGGGCGAGCGCCCACCC

Hpall
CCTACTTCACCTATCCTGCCCGG-3'
GGATGAAGTGGATAGGACGGGCC-5'

Figure 2

EP 0 528 925 B1

Figure 3

Figure 4